# EUROPEAN PATENT APPLICATION

(11) **EP 1 454 594 A1**
(43) Date of publication of application: **08.09.2004**
(21) Application number: 04004970.2
(22) Date of filing: 03.03.2004
(51) Int. Cl.: A61B 19/04

(54) **Thin wall gloves that release chlorine dioxide**

(30) Priority: 05.03.2003 US 451662 P; 05.03.2003 US 451663 P; 12.12.2003 US 733366
(71) Applicant: Microflex Corporation, Reno, Nevada 89523 (US)
(72) Inventor: Tao, Jian, Reno, Nevada 89523 (US); Peng, Shuo, Reno, Nevada 89501 (US)
(74) Representative: Fritz, Edmund Lothar, Dipl.-Chem.

(57) **Abstract**

Chlorine dioxide is a well-known disinfectant widely used in many applications. This invention discloses procedures and methodologies to make thin wall gloves that release chlorine dioxide gas. The resulting gloves have better barrier integrity. They are particularly efficient to prevent cross contamination, and the gloves preserve all the mechanical and physical properties of conventional.

## Description

The instant application is based upon U.S. Provisional Patent Application Serial No. 60/451,622, filed March 5, 2003 and U.S. Provisional Patent Application Serial No. 60/451,663, also filed March 5, 2003.

### BACKGROUND OF THE INVENTION

The present invention relates to thin wall gloves that release bacteria killing chlorine dioxide. The thin wall gloves, embodying the present invention, are produced with excellent barrier integrity. The thin wall gloves prevent cross-contamination, while preserving all the desired mechanical and physical properties of conventional gloves.

To protect users and to avoid cross contamination between things and people we need to touch, all sorts of protective equipment have been invented and are utilized in our daily life. Gloves have been the most common protective equipment for hand protection. Over the years, gloves have been made from variety of materials, plastics (polyvinyl chloride, polyethylene), rubbers (natural rubber latex, carboxylated polyacrylonitrile butadiene, polyurethane, polybutyl rubber, polyisoprene, polychloroprene), and thermal plastic elastomers (styrene-isoprene-styrene, styrene-ethylene-butadiene-styrene). There are gloves made from a combination of these materials, as well as blends or copolymers thereof. They all have different properties and therefore are suitable for different applications. Other than polyethylene (extrusion is needed), pretty much all the materials can be made into a dippable compound (aqueous or organic). The gloves are made via a dipping procedure.

Chlorine dioxide is an extremely active oxidant. It almost will react immediately with anything it is in touch with. Therefore, it is a highly dangerous gas if the concentration is high. But below a certain limit, as specified by OSHA and the EPA, it is an excellent disinfectant. As a matter of fact, it can be used to decontaminate the environment. It is also widely used in general cleaning products such as mouthwash liquid.

There are publications, which teach how to generate chlorine dioxide and use it in different applications safely. However, a glove that can generate chlorine dioxide has not yet been known.

The present invention contemplates a new and improved glove which overcomes the above-referenced problems and others.

### SUMMARY OF THE INVENTION

In accordance with this invention, a thin wall glove is provided that is capable of releasing chlorine dioxide. The thin wall glove has substantial bacteria killing efficacy. The thin wall glove can be made of polyurethane, carboxylated polyacrylonitrile butadiene, commonly known as nitrile, and or polyvinyl chloride. Methodologies for making a thin wall glove are also disclosed. The demonstrated procedures can be easily transferred to almost all the materials that can be used as glove making materials via an aqueous dipping process: including, but not limited to, natural rubber latex, polychloroprene, polyisoprene, polybutadiene, polybutyl rubber, and their copolymers and blends. The gloves are made of an organic solvent-based material such as a thermal plastic elastomer, or the gloves can have these materials as coatings on gloves.

### BRIEF DESCRIPTION OF THE DRAWINGS

**FIGURE 1** is a graphical representation of Bacteria killing efficacy as a measure of bacteria remaining vs. time (nitrile film);

**FIGURE 2** is a graphical representation of chlorine dioxide releasing activity upon light exposure (nitrile film);

**FIGURE 3** is a graphical representation of chlorine dioxide releasing activity upon light exposure (polyurethane film);

**FIGURE 4** is a graphical representation of chlorine dioxide releasing activity upon light exposure (polyvinyl chloride film);

**FIGURE 5** is a graphical representation of ASTM required physical properties, with and without disinfectant (nitrile film) ;

**FIGURE 6** is a graphical representation of ASTM required physical properties, with and without disinfectant (polyurethane film); and

**FIGURE 7** is a graphical representation of ASTM required physical properties, with and without disinfectant (polyvinyl chloride film).

### DETAILED DESCRIPTION OF INVENTION

Sodium chlorite is commonly used as a source of chlorine dioxide. When exposed to heat, light, moisture, and acid, sodium chlorite will release chlorine dioxide. Naturally, the releasing activity depends on environmental conditions.

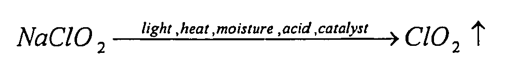

Sodium chlorite is incorporated into the gloves, and then, the gloves will release the desired chlorine dioxide.

First of all, the disinfectant is released while the gloves are being used. Otherwise, the designed purpose will not be served. A light activation mechanism is utilized. When sodium chlorite and titanium dioxide are mixed together, sufficient light exposure will cause sodium chlorite to decompose to release chlorine dioxide efficiently. In other words, titanium dioxide can act as the catalyst under light exposure.

Under the normal production and storage conditions, sodium chlorite is fairly stable. Most of the loaded sodium chlorite will be preserved. Upon its usage, the gloves are pulled out of storage box and exposed to light, the loaded sodium chlorite will start to decompose and release chlorine dioxide.

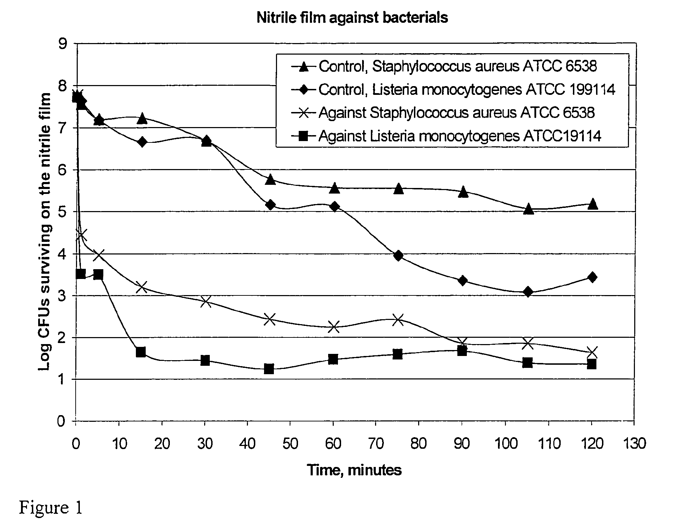

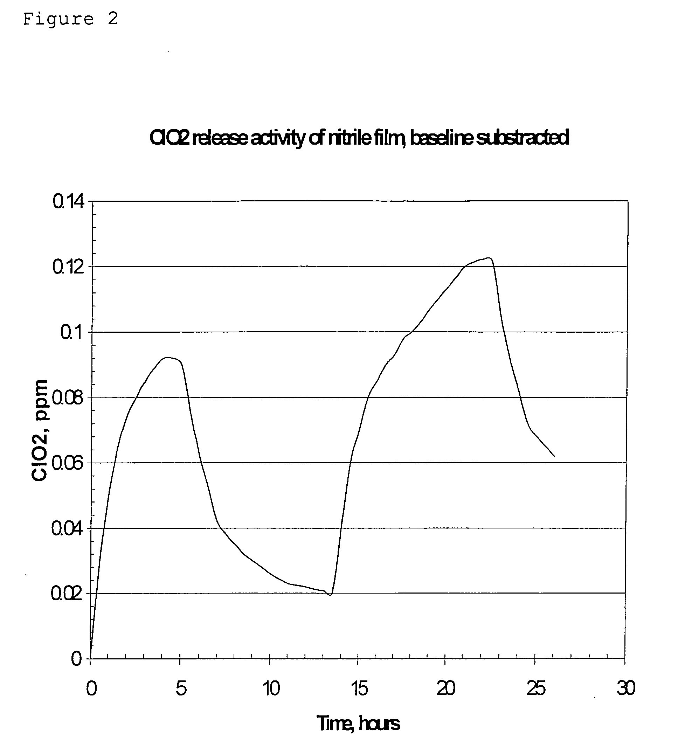

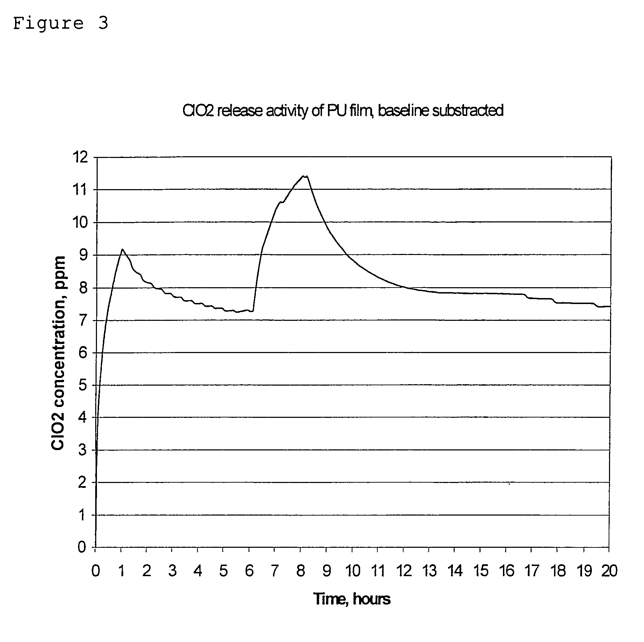

Secondly, releasing of disinfectant must be able to sustain at an efficacious level for a period of time to cover the typical usage span of a glove. At the same time, the released amount of disinfectant must not exceed the safety limit set by regulatory organizations such as OSHA. By controlling the amount of sodium chlorite loading, the efficacy of the film is maintained for up to 2 hours (see Figure 1). Figure 2, 3, and 4 show the accumulative concentration of chlorine dioxide is low and safe.

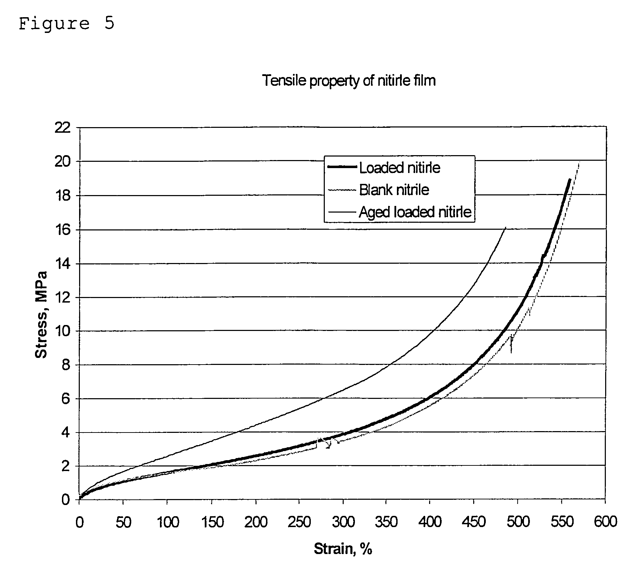

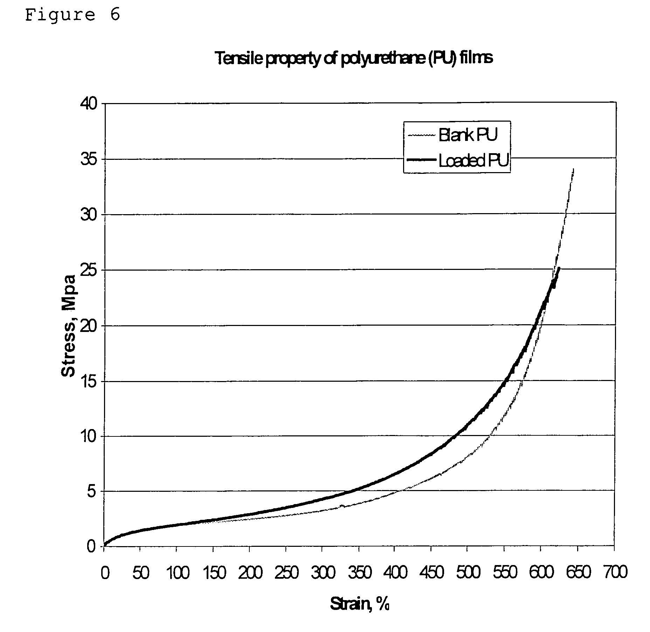

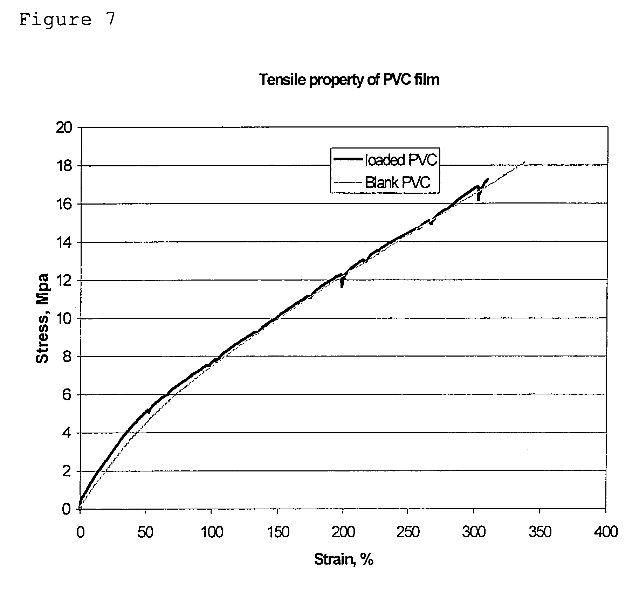

Thirdly, due to the fact that sodium chlorite is inorganic, and whereas all the materials for a gloves matrix are organic polymers, there are a series of challenges. Figure 5, 6, and 7 show the inventive films meet and exceed ASTM requirements for thin film gloves.

The aqueous medium selected for producing the thin wall glove include most rubbery materials: *i.e.*, natural rubber latex, carboxylated polyacrylonitrile butadiene, polyurethane, polybutyl rubber, polyisoprene, polychloroprene, polybutadiene. On the other hand, the organic media covers the plastic and the thermal plastic elastomer family as well, namely, polyvinyl chloride, styrene-isoprene-styrene, styrene-ethylene-butadiene-styrene, styrene-propylene-styrene, styrene-butadiene-styrene, etc.

Different materials have different formulations. Sodium chlorite is added in an amount so that it will not interfere with or be disabled in the final formulation.

Hence, several methods and procedures have been developed. Some materials can adapt to more than one method or procedure. To illustrate the methods and procedures, films were made via both an aqueous medium (polyurethane and carboxylated polyacrylonitrile butadiene) and an organic medium (polyvinyl chloride).

### Polyurethane

Traditionally, polyurethane used as elastic material is so called aliphatic polyurethane. It possesses excellent physical properties: high tensile strength and elongation, low modulus, exceptional puncture strength, *etc*. And the emulsion form of aliphatic polyurethane is commercially available. It is almost ideal for thin wall glove via a dipping procedure. However, it has not yet been widely used in disposable gloves because its high cost. Recently, an aromatic polyurethane emulsion was developed and a medical examination glove was commercialized. Although the performance is not as good as that of aliphatic polyurethane, its cost has been reduced to a reasonable level.

Due to the nature of polyurethane (very stable emulsion, no curing package needed, saturated polymer backbone), most complications in glove formation will not occur. Polyurethane is aqueous based and the sodium chlorite incorporated therein is water-soluble. Sodium chlorite is dissolved into a polyurethane dipping compound. Both compounding and dipping are straightforward.

As shown in equation 1, water, especially in acidic conditions, will initiate the decomposition of sodium chlorite. However, the solution of sodium chlorite itself is somewhat already basic. The final pH of dipping compound is controlled and is basic.

Essentially, the current set up for polyurethane glove manufacturing is adequate. The only requirement, and it is critical, is to keep light out throughout each and every stage of production, compounding, dipping, curing, former releasing, and packaging. It is impractical to conduct production in dark. Minimal light exposure is required. If light exposure is absolutely necessary, red light is recommended because longer wavelength light has little chance to trigger to the sodium chlorite decomposition. This is required for all the materials.

### Carboxylated polyacrylonitrile butadiene

Carboxylated polyacrylonitrile butadiene, or known commonly as nitrile, is the most widely used synthetic rubbery material in the thin wall glove industry as an alternative to natural rubber latex.

A nitrile dipping compound is an aqueous system that is much more delicate than polyurethane. A multiple components curing package must be used. Subtle changes in pH, solid content, temperature, viscosity, ionic strength, and so on, will result in destabilization of the emulsion system. Sodium chlorite was incorporated into nitrile films in following ways:

• Directly mixing sodium chlorite into dipping compound;

• Sandwich sodium chlorite between nitrile layers;

• Mixing sodium chlorite into polyurethane and use polyurethane as coating material;

• Two or all above approaches can be combined.

Some or all of these developed methodologies and in compounding and dipping can be easily transferred to other materials in aqueous systems, these include but are not limited to natural rubber latex, polybutyl rubber, polyisoprene, polychloroprene, polybutadiene, *etc*.

### Directly mixing sodium chlorite into dipping compound

Dumping a sodium chlorite powder into the latex compound does not work. The compound coagulated immediately. Before the powders dissolved, they have huge surface area. They will grab enormous amount of surfactants from emulsion droplets. The polymeric droplets would have to become bigger and bigger, and eventually become destabilized.

Dissolving the sodium chlorite first and then dumping the solution into the compound also failed. When certain amounts of sodium chlorite dissolve into the aqueous latex compound, the ionic strength balance of original emulsion will be off. Even if the compound can survive this process, it is still inferior because the sodium chlorite is going to start to decompose slowly in water, as aforementioned in the equation 1.

To get around the problem of dissolving the sodium chlorite into a potassium hydroxide solution, one has to use a concentration between 0.1 ∼ 5.0 %. The potassium hydroxide solution is a standard solution routinely used in latex dipping compound mixing for pH adjustment. Under basic conditions, the sodium chlorite is stable. The resulted solution has to be added slowly under stirring. Otherwise, one can still have local regions with high ionic strength and/or pH to shock the emulsion.

An advantage of this process is that the gloves could be produced in any factory. No major production line retrofit is needed other than light control.

### Sandwich sodium chlorite between nitrile layers

Typically, a glove is made following this sequence:

Coagulant (calcium nitrate), latex compound, pre leaching, vulcanization, post leaching

This is the so called single dipping procedure. Now, with the development of technology, double dipping becomes increasingly popular. Here is the dipping sequence:

Coagulant (calcium nitrate), latex compound, coagulant (calcium nitrate), latex again, pre leaching, vulcanization, post leaching

Sodium chlorite can be added into the second coagulant. Here is the disclosed sequence:

Coagulant (calcium nitrate), latex compound, coagulant (calcium nitrate + sodium chlorite), latex again, pre leaching, vulcanization, post leaching

The major advantage of this approach is that the danger of destabilizing the emulsion system is essentially eliminated and that the added sodium chlorite can be preserved better in the leaching stages.

While testing our films made this way, we noticed that no delamination was observed when the films were being stretched, as was a concern prior to our development work.

### Mixing sodium chlorite into polyurethane and using polyurethane as coating material

Polyurethane is commonly used in thin wall glove dipping manufacturing as a former releasing agent (mixed with coagulant) and coating material (one more additional dipping) to improve donning.

As previously discussed, the mixing of sodium chlorite and polyurethane is fairly straightforward; it is logical to adopt polyurethane as a carrier of disinfectant. Other carriers for the oxidant are contemplated such as: nitrile, natural rubber latex, polyisoprene, polychloroprene, polybutyl rubber, polybutadiene, polyurethane, polyvinyl chloride; carboxylated polyacrylonitrile butadiene; styrene-isoprene-styrene; styrene-ethylene-butadiene-styrene; styrene-propylene-styrene; and styrene-butadiene-styrene. The trouble of destabilizing the dipping compound is eliminated. And leaching is not a problem for loss of sodium chlorite, either. In the case of polyurethane mixed in coagulant as a former releasing agent, sodium chlorite is inside the hydrophobic film. In the case of the polymer coating, the dipping can be applied after leaching stages.

### Polyvinyl chloride

Both thermal plastic elastomers and polyvinyl chloride are chemically more stable than previous discussed systems. There is no need for chemical vulcanization packages. Thermal plastic elastomers are crosslinked via physical manners, so called micro phase separation. Polyvinyl chloride is a plastic. It shows mechanical strength that is acceptable without crosslinking. Therefore, one does not have to worry about destabilization of the dipping compounds.

In the case of polyvinyl chloride, two methods were developed to make gloves that release chorine dioxide:

• Directly mixing sodium chlorite into dipping compound

• Mixing sodium chlorite into polyurethane and use polyurethane as coating material.

A sandwich method does not work here because the film formation mechanism is entirely different from that of aqueous systems previously discussed. In previous systems, sodium chlorite solution can act as coagulant to form the film. However, polyvinyl chloride is formed via heat in contrast to chemically destabilized emulsification. The first layer of polyvinyl chloride and the sodium chlorite will change the heat conduction form former to plastisol dramatically. Therefore, multiple dipping of polyvinyl chloride plastisol is not an option.

Direct mixing sodium chlorite into polyvinyl chloride dipping plastisol works because sodium chlorite is not soluble in plasticizers, but it can be dispersed into it. The finer the inorganic particle size, results in a higher releasing efficiency of chlorine dioxide.

However, no matter how fine the particle size can be, it is not comparable with a water solution, dispersed at molecular level. The fact that sodium chlorite is not soluble in the dipping compound also causes quality problems for production. Even with help of certain dispersing agents, a thermodynamically homogenous dispersion is not achievable. Mechanical stirring also helps, but its effect is quite limited. As a result of all these factors, chemical sedimentation, which in turn results in quality inconsistency, is the major disadvantage of this approach.

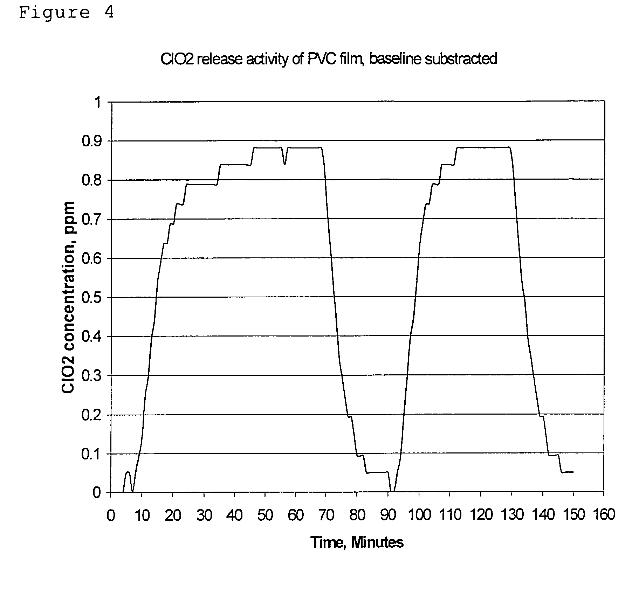

Incorporation of sodium chlorite into polyurethane is almost effortless. Polyurethane is commonly used as coating materials to improve donnability. As shown in Figure 4, the films made showed decent chlorine dioxide releasing activity.

The invention has been described with references to the preferred embodiments. Modifications and alterations will occur to others upon reading and understanding the preceding detailed description. It is intended that the invention be constructed as including all such modifications and alterations insofar as they come within the scope of the appended claims or the equivalents thereof.

## Claims

1. A thin walled medical glove that releases a strong oxidant as a disinfectant.

2. The thin walled glove as claimed in claim 1, wherein the glove is made from nitrile, natural rubber latex, polyisoprene, polychloroprene, polybutyl rubber, polybutadiene, polyurethane, polyvinyl chloride; carboxylated polyacrylonitrile butadiene; polybutyl rubber, styrene-isoprene-styrene; styrene-ethylene-butadiene-styrene; styrene-propylene-styrene; styrene-butadiene-styrene; or blends thereof.

3. The thin walled glove as claimed in claim 1, wherein the strong oxidant is chlorine dioxide.

4. The thin walled glove as claimed in claim 2, wherein the strong oxidant is chlorine dioxide.

5. The thin walled glove as claimed in claim 1, wherein the strong oxidant is released by exposure to light.

6. The thin walled glove as claimed in claim 1, wherein the strong oxidant is incorporated into the glove during formation as sodium chlorite.

7. The thin walled glove as claimed in claim 6, further comprising a catalyst incorporated therein for releasing the strong oxidant.

8. The thin walled glove as claimed in claim 7, wherein the catalyst is titanium dioxide.

9. The thin walled glove as claimed in claim 1, wherein the strong oxidant is contained in a coating present on the thin walled medical glove.

10. The thin walled glove as claimed in claim 9, wherein the coating is nitrile, natural rubber latex, polyisoprene, polychloroprene, polybutyl rubber, polybutadiene, polyurethane, polyvinyl chloride; carboxylated polyacrylonitrile butadiene; styrene-isoprene-styrene; styrene-ethylene-butadiene-styrene; styrene-propylene-styrene; styrene-butadiene-styrene; or blends thereof and sodium chlorite.

11. The thin walled glove as claimed in claim 9, wherein the coating comprises polyurethane and sodium chlorite.
